# EUROPEAN PATENT SPECIFICATION

(11) **EP 2 873 395 B1**
(45) Date of publication and mention of the grant of the patent: **30.03.2016**
(21) Application number: 13193365.7
(22) Date of filing: 18.11.2013
(51) Int. Cl.: A61F 5/02

(54) **Posture loop**
Haltungsschleife
Orthèse de posture

(43) Date of publication of application: 20.05.2015
(73) Proprietor: Chen, Paul, Vancouver, BC V7A 1S5 (CA)
(72) Inventor: Chen, Paul, Vancouver, BC V7A 1S5 (CA)
(74) Representative: Beck & Rössig European Patent Attorneys

(56) References cited:
- JP-A- 2009 178 539
- US-A- 1 581 791
- US-A- 5 259 833

## Description

The invention relates to a posture loop for straightening the shoulder and/or the back portion of a user as defined in the preamble portion of claim 1.

A posture loop of this type is known from US 1,581,791. This posture loop includes a cable and a bridge forming together an eight-shaped structure that is to be arranged around the shoulder and the back portion of a person. The bridge is stiffened with lateral and central bars 2, 3 of whale bone or the like. These bars 2, 3 are embedded in a textile sheath 1, 1'. Two eye parts 8, 8' for engaging with the ends of the cable are connected to the textile sheath 1, 1' by means of cord loops and traveller members.

Another posture loop is known from JP 2009-198539 A, which, however requires two upper cables and one lower cable. The free ends of these cables are connected with each other via buckles while the upper cables are connected to a bridge by means of a triangular eye part.

US 4,852,874 discloses a typical elastic exercise device comprising an eight (8)-shaped elastic rope or cable for exercising the muscle groups of the user.

However, the typical elastic exercise devices may not be used to straighten the shoulder and/or the back portion of the user.

The invention provides an alternative posture loop as defined in the claims.

The posture loop includes an elastic band or cable supported with a retainer bridge for forming an eight (8)-shaped structure and for straightening the shoulder and/or the back portion of the user.

In the drawing:
FIG. 1 is a rear plan schematic view of a posture loop;
FIG. 2 is a partial perspective view of the posture loop;
FIG. 3 is a partial exploded view of the posture loop;
FIG. 4 is a plan view of the posture loop;
FIG. 5 is an enlarged partial perspective view; and
FIG. 6 is a partial top plan view of the posture loop.

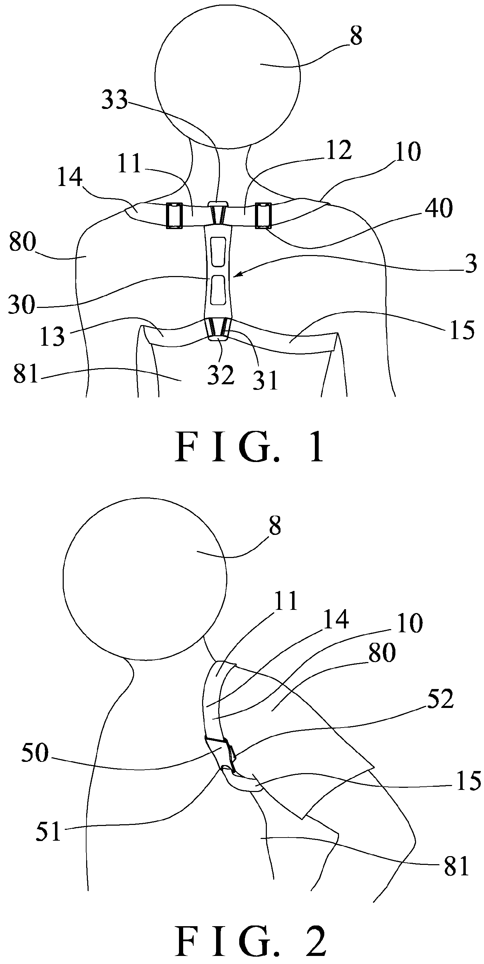

Referring to FIGS. 1-4, a posture loop comprises an elastic band or cable 10 having two ends 11, 12 to be coupled together for forming an endless loop, and having a middle portion 13 engaged with a retainer bridge 3 for forming an eight (8)-shaped structure and for engaging with the shoulders 80 of a user 8 (FIGS. 1-2) and for straightening the shoulder 80 and/or the back portion 81 of the user 8, or for correcting the posture of the user 8.

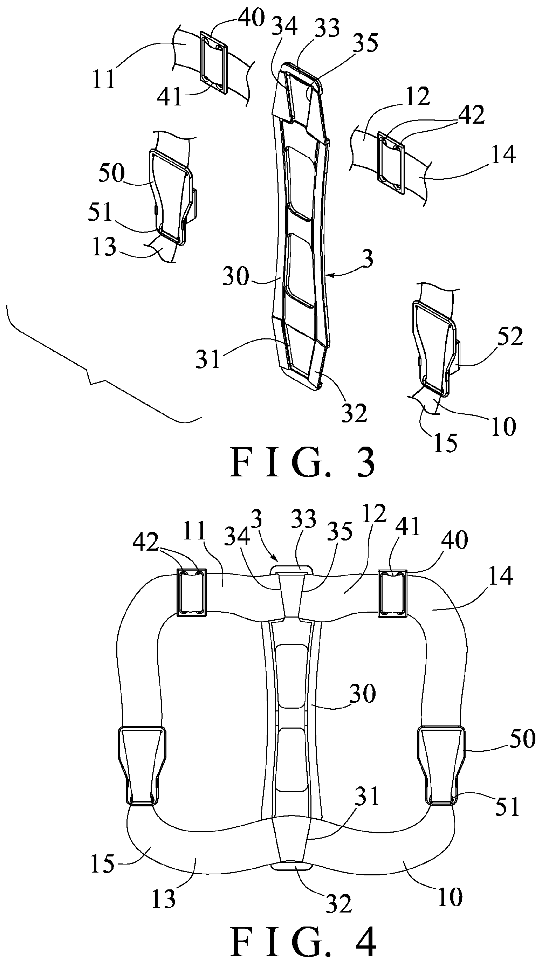

The bridge 3 (FIGS. 3-6) includes a bar 30 having a pathway 31 formed in the first end 32 for engaging with the middle portion 13 of the cable 10, the bar 30 includes a second end 33 having two channels 34, 35 for adjustably engaging with the ends 11, 12 of the cable 10, two buckles 40 each include a rib 41 for forming two grooves 42 and for engaging with the ends 11, 12 of the cable 10.

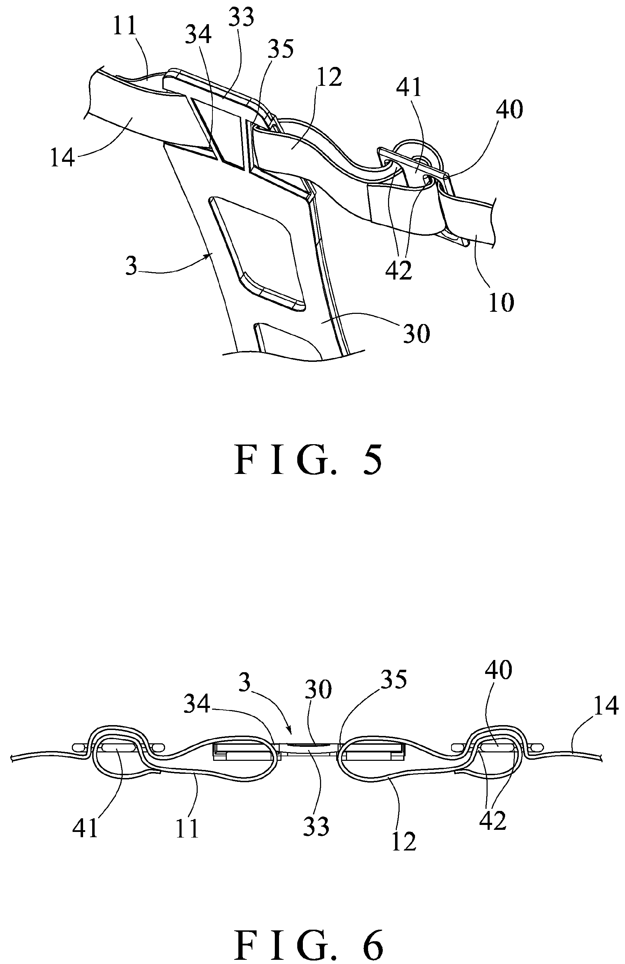

The ends 11, 12 of the cable 10 are engaged through the grooves 42 and over the rib 41 of the buckle 40 (FIGS. 5-6), and through the channel 34, 35 of the bar 30, and coupled to the rib 41 of the buckle 40 for adjustably coupling the ends 11, 12 of the cable 10 to the second end 33 of the bar 30 and for allowing the posture loop to be engaged with the shoulders 80 and/or the back portions 81 of the users 8 of different sizes or dimensions.

Two cushions 50 each include a passage 51 for slidably engaging with the middle portion 13 of the cable 10, and each include a pad 52 made of soft or resilient materials for softly engaging with the user 8.

The cable 10 is separated by the cushions 50 into a first segment 14 having the two ends 11, 12 of the cable 10, and a second segment 15 having most of the middle portion 13 of the cable 10.

The bridge 3 may separate the segments 14, 15 of the cable 10 from each other for forming the eight (8)-shaped structure.

In operation, the posture loop may be engaged the shoulders 80 and/or the back portion 81 of the user 8 (FIGS. 1-2) for exercising the shoulder 80 and/or the back portion 81 of the user 8. The ends 11, 12 of the cable 10 are adjustable relative to the second end 33 of the bar 30 of the bridge 3.

The tension of the cable 10 is supported via the cushions against a front portion of the user's body, preferably between the shoulder and armpit (axilla), respectively, while the bridge extends vertically along the back.

## Claims

1. A posture loop for straightening the shoulder and/or the back portion of a user, said posture loop comprising:
an elastic cable (10) including a first end (11), a second end (12), and a middle portion (13),
a bridge (3) including a bar (30) having a first end (32) and a second end (33), wherein the first end (11), the second end (12) and the middle portion (13) of the cable (10) are each engaged with the bridge (3) thereby forming an eight-shaped structure for engaging with the shoulders of the user, and
two buckles (40) attached to the first and the second ends (11, 12) of the cable (10) for allowing the posture loop to be adjusted to the shoulders and/or the back portions of users of different size or dimension,
**characterised in that**
the bar (30) includes two channels (34, 35) formed in the second end (33) of the bar (30) and slidably engaged with the first and the second ends (11, 12) of the cable (10), respectively, and
the middle portion (13) of the cable (10) is engaged with the first end (32) of the bar (30).

2. The posture loop as claimed in claim 1, wherein the bar (30) includes a pathway (31) formed in the first end (32) of the bar (30) for slidably engaging with the middle portion (13) of the cable (10).

3. The posture loop as claimed in claim 1 or 2, wherein the buckles (40) each include a rib (41) for forming two grooves (42) and for engaging with the first and the second ends (11, 12) of the cable (10) respectively.

4. The posture loop as claimed in one of claims 1 to 3 further comprising two cushions (50) each including a passage (51) for engaging with the middle portion (13) of the cable (10).

5. The posture loop as claimed in claim 4, wherein the cable (10) is separated into a first segment (14) and a second segment (15) with the cushions (50), and the bar (30) is engaged between the segments (14, 15) of the cable (10).

## Patentansprüche

1. Haltungsschleife zum Aufrichten der Schulter und/oder des Rückenteils eines Benutzers, wobei die Haltungsschleife umfasst:
ein elastisches Band (10) mit einem ersten Ende (11), einem zweiten Ende (12) und einem Mittelabschnitt (13),
eine Brücke (3) mit einem Stab (30) mit einem ersten Ende (32) und einem zweiten Ende (33), wobei das erste Ende (11), das zweite Ende (12) und der Mittelabschnitt (13) des Bandes (10) jeweils mit der Brücke (3) in Eingriff stehen und dadurch eine achtförmige Struktur zum Umschließen der Schultern des Benutzers bilden, und
zwei Schnallen (40), die an dem ersten und zweiten Ende (11, 12) des Bandes (10) befestigt sind und das Anpassen der Haltungsschleife an die Schultern und/oder den Rückenteil von Benutzern mit unterschiedlicher Größe und Maßen ermöglichen,
**dadurch gekennzeichnet, dass**
der Stab (30) zwei Kanäle (34, 35) aufweist, die in dem zweiten Ende (33) des Stabes (30) ausgebildet sind und jeweils verschiebbar mit dem ersten bzw. zweiten Ende (11, 12) des Bandes (10) in Eingriff stehen, und
der Mittelabschnitt (13) des Bandes (10) mit dem ersten Ende (32) des Stabes (30) in Eingriff steht.

2. Haltungsschleife nach Anspruch 1, wobei der Stab (30) einen Durchgang (31) aufweist, der in dem ersten Ende (32) des Stabes (30) ausgebildet ist, um verschiebbar mit dem Mittelabschnitt (13) des Bandes (10) in Eingriff zu kommen.

3. Haltungsschleife nach Anspruch 1 oder 2, wobei die Schnallen (40) jeweils eine Rippe (41) aufweisen, um zwei Schlitze (42) zu bilden und um mit dem ersten bzw. zweiten Ende (11, 12) des Bandes (10) in Eingriff zu kommen.

4. Haltungsschleife nach einem der Ansprüche 1 bis 3, des Weiteren umfassend zwei Polster (50), die jeweils einen Durchgang (51) aufweisen, um mit dem Mittelabschnitt (13) des Bandes (10) in Eingriff zu kommen.

5. Haltungsschleife nach Anspruch 4, wobei das Band (10) mit den Polstern (50) in ein erstes Segment (14) und ein zweites Segment (15) unterteilt ist, und der Stab (30) zwischen den Segmenten (14, 15) des Bandes (10) in Eingriff steht.

## Revendications

1. Orthèse de posture pour redresser l'épaule et/ou la portion de dos d'un utilisateur, ladite orthèse de posture comprenant :
un câble élastique (10) qui comprend une première extrémité (11), une seconde extrémité (12) et une portion du milieu (13),
un pont (3) qui comprend une barre (30) qui a une première extrémité (32) et une seconde extrémité (33), la première extrémité (32), la seconde extrémité (33) et la portion du milieu (13) du câble (10) étant chacune engagée avec le pont (3) en formant ainsi une structure en forme de huit pour être en prise avec les épaules de l'utilisateur et
deux boucles (40) attachées à la première et à la seconde extrémité (11, 12) du câble (10) pour permettre à l'orthèse de posture d'être ajustée aux épaules et/ou aux portions de dos d'utilisateurs de différentes tailles ou dimensions,
**caractérisée en ce que**
la barre (30) comprend deux canaux (34, 35) formés dans la seconde extrémité (33) de la barre (30) et en prise par coulissement respectivement avec la première et la seconde extrémité (11, 12) du câble (10) et la portion du milieu (13) du câble (10) est en prise avec la première extrémité (32) de la barre (30).

2. Orthèse de posture selon la revendication 1, dans laquelle la barre (30) ayant un passage (31) formé dans la première extrémité (32) de la barre (30) pour mettre en prise de manière coulissante avec la partie médiane (13) du câble (10).

3. Orthèse de posture selon la revendication 1 ou 2, dans laquelle les boucles (40) comprennent chacune une nervure (41) pour former deux rainures (42) et pour mettre en prise avec les première et seconde extrémités (11, 12) du câble (10), respectivement.

4. Orthèse de posture selon l'une quelconque des revendications 1 à 3, comprenant en outre deux coussins (50) comportant chacun un passage (51) pour mettre en prise avec la partie médiane (13) du câble (10).

5. Orthèse de posture la revendication 4, dans laquelle le câble (10) est séparé en un premier segment (14) et un deuxième segment (15) avec les coussins (50), et la barre (30) est engagé entre le les segments (14, 15) du câble (10).
